## Europäisches Patentamt

## European Patent Office

## Office européen des brevets

(19)

(11) Numéro de publication: **0 252 894**
A2

(12)

# DEMANDE DE BREVET EUROPEEN

(21) Numéro de dépôt: 87870070.7

(22) Date de dépôt: 13.05.87

(51) Int. Cl.⁴: **C 12 N 15/00**
C 12 P 21/02, C 12 N 1/20
//(C12N1/20,C12R1:19)

(30) Priorité: 15.05.86 US 863281

(43) Date de publication de la demande:
13.01.88 Bulletin 88/02

(84) Etats contractants désignés:
AT BE CH DE ES FR GB GR IT LI LU NL SE

(71) Demandeur: Smith Kline - RIT Société anonyme dite:
rue du Tilleul, 13
B-1320 Genval (Rixensart) (BE)

(72) Inventeur: Bollen, Alex Joseph
Gaasbeekstraat, 68
B-1711 Itterbeek (BE)

Jacobs, Paul
Chemin des Crolithes, 119
B-7806 Ianquesaint (BE)

(74) Mandataire: Tasset, Gérard
SMITHKLINE - RIT rue de l'Institut, 89
B-1330 Rixensart (BE)

(54) Polypeptides hybrides comprenant la somatocrinine et l'alpha1-antitrypsine, méthode pour leur production à partir de clones bactériens et leur utilisation pour la production de somatocrinine.

(57) On utilise une fusion entre séquences d'ADN codant pour hAT et hGRF par l'intermédiaire d'un adaptateur de synthèse codant pour une séquence d'acides aminés clivable in vitro pour exprimer hGRF à des taux élevés dans E. coli.

EP 0 252 894 A2

## Description

Polypeptides hybrides comprenant la somatocrinine et l'alpha$_1$-antitrypsine, méthode pour leur production à partir de clones bactériens et leur utilisation pour la production de somatocrinine

Domaine de l'invention

La présente invention concerne des polypeptides hybrides comprenant la somatocrinine et l'alpha$_1$-antitrypsine, une méthode pour leur production au départ de clones bactériens et leur utilisation pour la production de somatocrinine.

Plus précisément, la méthode et l'utilisation de cette invention consistent en une série d'étapes comprenant la liaison en phase, par l'intermédiaire d'un adaptateur ADN de synthèse codant pour une séquence d'amino acides clivable in vitro, d'une séquence de nucléotides correspondant à la somatocrinine, c'est-à-dire le facteur de libération de l'hormone de croissance humaine mature ou un fragment ou dérivé actif de celui-ci et au moins un fragment de la séquence codant pour l'alpha$_1$-antitrypsine humaine qui s'exprime elle-même de manière optimale dans E. coli, l'introduction des séquences liées dans un puissant vecteur d'expression bactérien. La production du polypeptide hybride et la scission in vitro dudit polypeptide pour donner la somatocrinine.

Arrière-plan technologique

Le facteur de libération de l'hormone de croissance humaine qui est également appelé somatocrinine (ci-après désigné par l'abréviation "hGRF") est un régulateur positif de la sécrétion de l'hormone de croissance (ci-après désignée par l'abréviation "GH") par l'adénohypophyse. L'hGRF a été isolé d'une tumeur pancréatique humaine causant une acromégalie; il est constitué d'un polypeptide de 44 acides aminés dont la séquence a été établie (GUILLEMIN et al., Science 218, 585-587, 1982). Des anticorps contre ce peptide ont permis l'identification d'une substance immunoréactive dans l'hypothalamus de différents primates et un polypeptide apparemment identique a également été isolé de l'hypothalamus humain. Enfin, des expériences de type méthode de transfert dite "Southern blot" indiquent qu'il n'existe qu'un seul gène codant pour hGRF. Ceci suggère donc que le facteur pancréatique tumoral est codé par le même mARN que le facteur hypothalamique physiologique. L'utilité de la faculté de produire des quantités substantielles de GRF humaine est, en particulier, due au fait que sa déficience génétique ou physiologique est une cause de dwarfisme, que sont effet stimulant sur la synthèse de l'hormone de croissance (GH) devrait amener à son utilité dans la diagnose de déficiences ou de désordres du métabolisme de la GH, que son administration pourrait provoquer une accélération de la régénération tissulaire, par exemple dans le traitement de victimes de graves brûlures et qu'il a été démontré que l'administration de GRF humaine à des animaux stimulait leur croissance.

Une forme d'hGRF n'ayant que 40 résidus d'acides aminés a également été signalée et des fragments de GRF n'ayant seulement qu'environ 27 résidus d'acides aminés ont été trouvés biologiquement actifs. Le peptide pancréatique hGRF complet avec ses 44 résidus d'acides aminés a été synthétisé par voie chimique et l'hGRF synthétique, les fragments d'hGRF synthétiques et leurs analogues synthétiques représentent une source potentielle de substances régulatrices très puissantes mais la synthèse chimique de chaînes peptidiques aussi longues est cependant très coûteuse.

Des techniques d'ADN recombinant par lesquelles l'ADN d'hGRF est introduit dans une cellule pour exprimer hGRF ont également été développées.

Néanmoins ces techniques ne permettent qu'une production plutôt limitée d'hGRF.

Le brevet belge 898.666 décrit une telle technique dans laquelle des fragments d'ADN synthétisés chimiquement sont combinés à des fragments d'ADN isolés de sources naturelles.

Le procédé de la présente invention est inspiré de la méthode qui consiste à produire des protéines de fusion ou hybrides à partir desquelles la protéine désirée elle-même est alors éventuellement récupérée.

Comme exemples d'une telle méthode on peut citer :

- la demande de brevet européen publiée sous le No. 0 020 290 et la demande de brevet allemand No. 2 922 496 qui décrivent la préparation d'une protéine par scission enzymatique sélective du C-terminal d'une protéine de fusion avec le N-terminal d'une séquence tétrapeptidique particulière;

- la demande de brevet européen publiée sous le No. 0 133 282 qui décrit la préparation d'une amide polypeptidique comme le facteur de libération de l'hormone de croissance par la production d'un peptide glycinique par technologie génétique et élimination de la glycine C-terminale par voie enzymatique;

- la demande de brevet australien No. 84/37071 qui décrit la préparation d'une protéine hybride fibrinolytiquement active par différentes méthodes parmi lesquelles la méthode comprenant la prise d'information génétique de chaque protéine, leur coupure et leur ligation pour construire une séquence d'ADN codant pour la protéine hybride et l'expression de cet ADN dans des hôtes prokaryotes ou eukaryotes, les chaînes séparées étant éventuellement préparées ensuite par réduction douce pour casser les ponts disulfure interchaînes suivie d'une chromatographie d'affinité;

- la demande de brevet européen publiée sous le No. 0 134 085 qui décrit la production du facteur de libération de l'hormone de croissance pancréatique pré-pro-humaine (hpGRF) par technique d'ADN recombinant, en utilisant une séquence nucléotidique codant pour l'entièreté de la séquence peptidique de hpGRF, un segment peptidique lié à la terminaison amino du peptide hpGRF et un segment peptidique lié à la terminaison carboxy du peptide hpGRF, ledit précurseur étant transformé en GRF lorsque des enzymes de traitement appropriées sont présentes dans les cellules transformées;
- la demande de brevet européen publiée sous le No. 0 129 073 qui décrit un procédé dans lequel un peptide présentant l'activité du facteur de libération de l'hormone de croissance est préparé par culture d'une levure transformée avec une construction d'ADN comprenant une séquence codante pour le facteur de libération de l'hormone de croissance et exprimant une protéine de fusion.

Résumé de l'invention

Suivant la présente invention, on prépare l'hGRF dans un clone bactérien transformé avec un vecteur plasmide recombinant comprenant une séquence d'ADN codant pour hGRF et une séquence d'ADN codant pour l'alpha$_1$-antitrypsine humaine (hAT) ou au moins un fragment de celle-ci renfermant les codons depuis l'acide aminé 2 environ ou l'acide aminé 15 environ jusqu'à l'acide aminé 363 environ, les deux séquences étant fusionnées en phase par l'intermédiaire d'un adaptateur ADN de synthèse portant l'information correspondant à la séquence d'acide aminé (Asp)$_4$Lys.

Le procédé de la présente invention peut être schématisé par la série d'étapes suivante :

(1) préparation d'une séquence d'ADN codant pour hGRF ou pour un fragment ou dérivé actif de celui-ci préparé par synthèse chimique totale ou par transcription inverse ou par combinaison des deux mais, de préférence, par synthèse chimique totale, de préférence en partant du codon pour l'acide aminé 1 du polypeptide;

(2) isolement d'un fragment d'ADN codant pour l'alpha$_1$-antitrypsine humaine préparément par synthèse chimique totale ou par transcription inverse ou par combinaison des deux, de préférence par transcription inverse et renfermant les codons depuis l'acide aminé 2 environ ou l'acide aminé 15 environ jusqu'à l'acide aminé 363 environ;

(3) préparation d'un adaptateur ADN de synthèse portant l'information correspondant à la séquence d'acides aminés (Asp)$_4$Lys qui est clivable in vitro par l'enzyme entérokinase et des sites de restriction appropriés pour la fusion en phase de séquences d'hAT et d'hGRF;

(4) fusion en phase et lecture de la terminaison 5' à la terminaison 3' d'une séquence codant pour hAT, d'un adaptateur ADN de synthèse codant pour une séquence d'acides aminés clivable in vitro et du gène hGRF, c'est à-dire dans l'ordre hAT-séquence clivable-hGRF;

(5) insertion de la séquence de fusion dans un puissant vecteur d'expression bactérien;

(6) transformation d'une bactérie E. coli hôte à l'aide du vecteur plasmide recombinant;

(7) sélection des bactéries transformées qui portent le plasmide recombinant;

(8) production du polypeptide hybride recombinant dans une bactérie E. coli transformée;

(9) clivage in vitro du polypeptide hybride produit dans les cellules de E. coli, par l'enzyme entérokinase;

(10) obtention d'hGRF mature et sa caractérisation par méthodes immunologiques.

Plus particulièrement et par exemple, cette série d'étapes comprend :

(1) l'obtention d'un fragment d'ADN de 135 pb codant pour hGRF par digestion de l'ADN d'un plasmide approprié (p. ex. pULB1113 décrit dans le brevet belge 898.666) avec les enzymes RsaI et SalI;

(2) l'obtention d'un fragment d'ADN de 1081 ou 1044 pb codant pour l'entièreté ou une partie de hAT, c'est-à-dire s'étendant de l'acide aminé 2 ou 15 jusqu'à 363, par digestion de l'ADN d'un plasmide approprié (p. ex. pULB1523 décrit dans le brevet belge 895.961) avec les enzymes BamHI ou BclI et AvaI;

(3) la synthèse d'un oligomère d'une longueur de 27 bases et d'un analogue complémentaire de 23 bases qui, par hybridation (annealing), constitue l'adaptateur ADN de synthèse codant pour la séquence d'acides aminés (Asp)$_4$Lys clivable in vitro par l'enzyme entérokinase et portant des sites de restriction appropriés;

(4) la ligation à l'aide d'ADN de ligase de T4 à l'adaptateur de synthèse des fragments d'ADN correspondant à hGRF et à hAT de manière à obtenir une fusion en phase;

(5) l'insertion des séquences d'ADN liguées dans l'ADN d'un puissant vecteur d'expression bactérien portant un promoteur thermoinductible (p. ex. l'ADN du plasmide pAS1).

(6) la transformation d'une bactérie lysogène (plus particulièrement une souche d'E. coli lysogène, par exemple la souche AR58 avec le plasmide de l'étape (5));

(7) sur base de la résistance antibiotique, la sélection des clones bactériens qui portent ledit plasmide;

(8) la production de polypeptide hybride dans une bactérie transformée en induisant le promoteur porté par le plasmide recombinant;

(9) le clivage du polypeptide hybride à l'aise d'entérokinase;

(10) la caractérisation par réaction avec des anticorps anti-hGRF de hGRF mature résultant du clivage du polypeptide hybride.

Description détaillée de l'invention

Il a maintenant été trouvé que l'expression d'hGRF dans les bactéries peut être améliorée d'une manière significative et inattendue en procédant à la fusion de la séquence codant pour hGRF à une séquence codante correspondant à hAT ou à un fragment de celle-ci qui s'exprime elle-même de manière optimale dans les bactéries, de telle manière que la direction à la transcription soit hAT vers hGRF.

En utilisant la molécule d'ADN recombinant de l'invention, hGRF est alors produit en grande quantité dans les bactéries alors qu'on ne détecte pas d'hGRF lorsque sa séquence correspondante d'ADN est clonée telle quelle dans un vecteur d'expression bactérien (CRAVADOR et al., Biochimie 67, 829, 1985)

Plus particulièrement, la séquence codante dans la molécule recombinante de l'invention comprend (1) le fragment de la séquence codant pour hAT et s'étendant du codon pour l'acide aminé 2 environ ou l'acide aminé 15 environ jusqu'à l'acide aminé 363 environ, (2) un nucléotide de synthèse codant pour le peptide (Asp)₄Lys qui est clivable in vitro par l'entérokinase en employant des techniques enzymologiques standard et (3) une séquence d'ADN pour hGRF partant du codon désignant l'acide aminé 1 de la molécule mature. Cette séquence a été obtenue par synthèse chimique standard comme décrit par CRAVADOR et al. (loc. cit.) et dans le brevet belge 898.666. Le vecteur plasmide d'expression utilisé dans l'invention emploie le promoteur de gauche (P_L) de lambda et le site de fixation ribosomique cII. La construction du plasmide recombinant est décrite dans l'exemple ci-dessous dans lequel les données d'expression ont été obtenues par induction thermique (Meth. Enzym. 101, 123, 1983). Le polypeptide hybride résultant comprend donc N-hAT-site de clivage enzymatique-hGRF-C-.

Il est évident que les molécules d'ADN recombinant décrites dans la présente spécification sont purement illustratives et non limitatives de l'invention. Des constructions alternatives peuvent être faites par des techniques bien connues dans le domaine de la génétique moléculaire. Les effets de ces constructions alternatives sur les taux d'expression dans les bactéries peuvent être facilement déterminés par des méthodes bien connues telles que ELISA comme décrite, par exemple, par BOLLEN et al. (DNA 2, 255, 1983). D'autres molécules d'ADN illustratives de l'invention sont celles dans lesquelles la dimension de la séquence codante hAT fusionnée à la séquence hGRF est plue courte que celle qui est décrite dans l'exemple ci-dessous ou dans lesquelles la séquence nucléotidique hGRF est modifiée sans modification de la séquence des acides aminés.

Le vecteur d'expression dans lequel la séquence d'ADN fusionnée codant pour hAT et hGRF est insérée pour préparer le vecteur de l'invention peut être l'un ou l'autre des nombreux vecteurs d'expression bactériens connus et accessibles à l'homme de l'art. Les vecteurs d'expression qui peuvent être régulés sont préférés. Dans cette spécification, l'expression 'pouvant être régule' signifie que la transcription de la séquence codante insérée n'est pas constitutive mais qu'elle peut être induite, par exemple par addition d'un agent d'induction au milieu de culture ou par la chaleur. Commes exemples de vecteurs d'expression d'E. coli on peut citer entre autres pCQV2 décrit par QUEEN, C. (J. Mol. Appl. Genet. 2, 1- 10, 1983) et pAS1 décrit par M. ROSENBERG et al. (Meth. Enzym. 101, 123-138, 1983). pAS1 porte l'origine pBR322 de réplication, un marqueur de résistance à l'ampicilline et une série de fragments de lambda qui comprennent la région de régulation, à savoir le promoteur de gauche de lambda (P₂), des sites de reconnaissance de la fonction N-anti-terminaison (NutL et NutR), le signal de terminaison de transcription rho-dépendant (tR1) et le site de fixation ribosomique cII, incluant le site d'initiation de translation cII, dont le résidu G est immédiatement suivi d'une site de clivage BamHI, comme suit

5′ .....CATATG*GATC.....3′

dans lequel le symbole * représente le site de clivage pour BamHI.

pAS1 peut être obtenu au départ de pKC30cII par élimination des nucléotides allant du site BamHI à la jonction cII-pBR322 de pKC30cII jusqu'à cII-ATG et en religuant la molécule pour régénérer le site BamHI immédiatement en aval de ATG. pKC30cII est construit en insérant un fragment HaeIII de 1,3 kb de lambda qui porte le gène cII dans le site HpaI de pKC30 (SHATZMAN et al., Experimental Manipulation of Gene Expression, édité par M. INOUYE, Academic Press, NewYork, 1983 et M. ROSENBERG et al. loc. cit.). pKC30 est décrit par SHIMITAKE et al. (Nature 292, 128, 1981). Il s'agit d'un dérivé de pBR322 qui a un fragment HindIII-BamHI de 2,4 kb de lambda inséré entre les sites HindIII et BamHI dans le gène tetR de pBR322. Des constructions similaires au pAS1 sont décrites par COURTNEY et al. (Nature 313, 149, 1985) et par KOTEWICZ et al. (Gene 35, 249, 1985). La séquence codante y est insérée par technique standard de manière opérative, c'est-à-dire dans une orientation correcte et dans un cadre de lecture approprié à l'élément régulateur.

D'autres systèmes de clonage et d'expression sont connus et accessibles pour exprimer la séquence codante de l'invention dans d'autres bactéries, p. ex. Bacillus, Streptomyces, Corynebacterium et autres.

Une séquence codante pout hAT mature, d'où sont dérivés les fragments d'ADN utilisés dans l'invention, peut être obtenue par des techniques standard par transcription inverse de populations sélectionnées d'ARN messager provenant de cellules hépatiques comme décrit, par exemple, par BOLLEN et al. (Brevet belge No. 895.961); BOLLEN et al. (DNA 2, 255, 1983); COURTNEY et al. (Demande de brevet européen publiée sous le No. 0 114 777); KAWASAKI et al. (The Molecular Biology of Yeast, Cold Spring Harbour Laboratory, 16-21 août, 1983); LONG et al. (Structure and Organization of Genes I, Cold Spring Harbour Laboratory, Abstract No. 25, 1983); WOO et al. (From Gene to Protein : Translation into Biotechnology, édité par AHMED et al., Academic Press, New-York, 1982); KURACHI et al. (Proc. Natl. Acad. Sci. USA 78, 6826, 1981); PARKER et al. (Demande de brevet australien 84/31801) et COSTANZO et al. (EMBO J. 2, 57, 1983), toutes ces citations étant données ici comme énoncées en détail suivant ces références.

Un ADN entièrement synthétique codant pour hGRF peut être obtenu par procédé standard bien connu dans la technique tel que décrit, par exemple, par CRAVADOR et al. (Brevet belge No. 898.666 et Biochimie 67, 829, 1985) ou par BARR et al. (Demande de brevet européen publiée sous le No. 0 129 073). Les polypeptides hybrides de l'invention sont des polypeptides hAT/hGRF pontés; ils sont produits par culture de microorganismes ou de cellules qui ont été transformées avec le vecteur d'expression de l'invention, dans des conditions permissives. Par "conditions permissives" on entend conditions de culture, p. ex. analytes, métabolites, autres ingrédients de culture et température, dans lesquelles l'expression de ladite protéine hybride est induite. Typiquement, des E. coli transformés sont cultivés dans un milieu nutritif contenant des sources assimilables de carbone et d'azote, avec aération et sous pression sélective jusqu'à développement en phase exponentielle de croissance ($A_{650}$ environ 0,4-0,6) avant induction et ensuite pendant une période additionelle de 1-1/2 à 5 heures après induction jusqu'à ce qu'une quantité de polypeptide récupérable soit exprimée.

Les polypeptides hybrides de l'invention exprimés dans E. coli ou autres microorganismes sont isolés de la culture productrice par des techniques standard d'isolement de protéine.

Typiquement, le schéma de purification comprend (1) la rupture des cellules, (2) la clarification de l'extrait cellulaire, (3) la séparation du polypeptide hybride hAT/hGRF et (4) la purification finale pour éliminer les contaminants. Le premier stade peut être réalisé, par exemple, par addition de lysozyme ou autre agent de lyse ou de perméabilisation ou par rupture mécanique ou ultrasonique. D'autres moyens pour libérer le polypeptide comprennent l'utilisation de bactéries lytiques thermosensibles, comme décrit par AUERBACH et ROSENBERG (Demande de brevet européen publiée sous le No. 0 140 864). Lorsque le polypeptide hybride est précipité dans l'extrait cellulaire, il peut être resolubilisé par addition d'un dénaturant. De telles techniques sont bien connues de l'homme de l'art. Plusieurs parmi elles ont été passées en revues par BUILDER et al. (Demande de brevet européen publiée sous le No. 0 114 506). Les molécules hybrides hAT/hGRF spécifiquement décrites ici sont solubles dans des extraits standard d'E. coli.

Les molécules hAT/hGRF pontées solubles de l'invention peuvent être séparées par des techniques standard.

Le clivage des molécules hAT/hGRF pontées de l'invention peut être effectué par digestion enzymatique avec l'enzyme entérokinase dans des conditions standard comme celles qui sont décrites par MAROUX et al. (J. Biochemistry 246, 5031, 1971). L'hGRF mature qui résulte du clivage peut alors être séparé par des méthodes de chromatographie standard telles que celles qui mettent en oeuvre des tamis moléculaires. Un procédé de purification préféré comprend une chromatographie d'affinité employant un anticorps polyclonal ou monoclonal d'hGRF fixé qui sépare l'hGRF clivé des molécules d'hAT.

Les polypeptides ainsi obtenus ou leurs sels non toxiques combinés à un excipient acceptable au point de vue pharmaceutique ou vétérinaire pour former une composition pharmaceutique peuvent être administrés à des humains ou à des animaux par voie intraveineuse, sous-cutanée, intramusculaire ou transdermale, par exemple par voie nasale ou même orale, comme il est bien connu dans la pratique. L'administration peut être employée pour stimuler la libération de GH (hormone de croissance) lorsque l'hôte qui est traité requiert un tel traitement thérapeutique. Le dosage requis variera en fonction de l'état pathologique particulier à traiter, de la gravité de l'état pathologique et de la durée du traitement désiré.

De tels peptides sont souvent administrés sous la forme de sels non toxiques tels que les sels d'addition avec les acides ou sous forme de complexe métallique, p. ex. avec le zinc, le fer ou un autre métal du même genre (lesquels sont considérés comme sels pour les buts de la présente description). Comme exemples illustratifs de tels sels d'addition avec les acides on peut citer le chlorhydrate, le bromhydrate, le sulfate, le phosphate, le maléate, l'acétate, le citrate, le benzoate, le succinate, le malate, l'ascorbate, le tartrate et d'autres du même genre. Quand l'ingrédient actif doit être administré oralement sous forme de comprimé, la comprimé peut contenir un liant, comme la gomme adragante, l'amidon de maïs ou la gélatine, un agent de désintégration comme par exemple l'acide alginique et un lubrifiant comme le stéarate de magnésium. Lorsqu'on désire une administration sous forme liquide, on peut utiliser un agent sucrant ou parfumé et pour une administration intraveineuse dans du sérum physiologique, on peut utiliser des solutions à base de tampon phosphate ou d'autres du même genre.

Les peptides sont à administrer à des humains sous contrôle médical et les compositions pharmaceutiques contiendront habituellement le peptide avec un excipient solide ou liquide conventionnel pharmaceutiquement acceptable. Habituellement, le dosage parentéral variera entre environ 0,01 et environ 1 microgramme de peptide par kilogramme de poids de l'hôte.

Dans l'exemple suivant qui - comme il est indiqué ci-avant - est illustratif et non limitatif de l'invention, toutes les endonucléases de restriction sont obtenues de sources commerciales et utilisées en substance en suivant les instructions du vendeur.

EXEMPLE

Stade 1. Le gène d'hAT humaine est isolé du clone cADNpULB1523 (décrit dans le brevet belge No. 895.961). Le gène est obtenu sur un fragment PstI contenant tout le clone cADN. Le fragment est mis à digérer avec les endonucléases de restriction BamHI et AvaI; il porte la séquence codant pour les acides aminés 2 à 363 de hAT.

Stade 2. L'hGRF humaine entièrement synthétique est isolée du clone pULB1113 (décrit dans le brevet belge No. 898.666) par digestion avec les enzymes des restriction RsaI et SalI. Le fragment d'ADN obtenu code pour hGRF mature commençant à l'acide aminé 1.

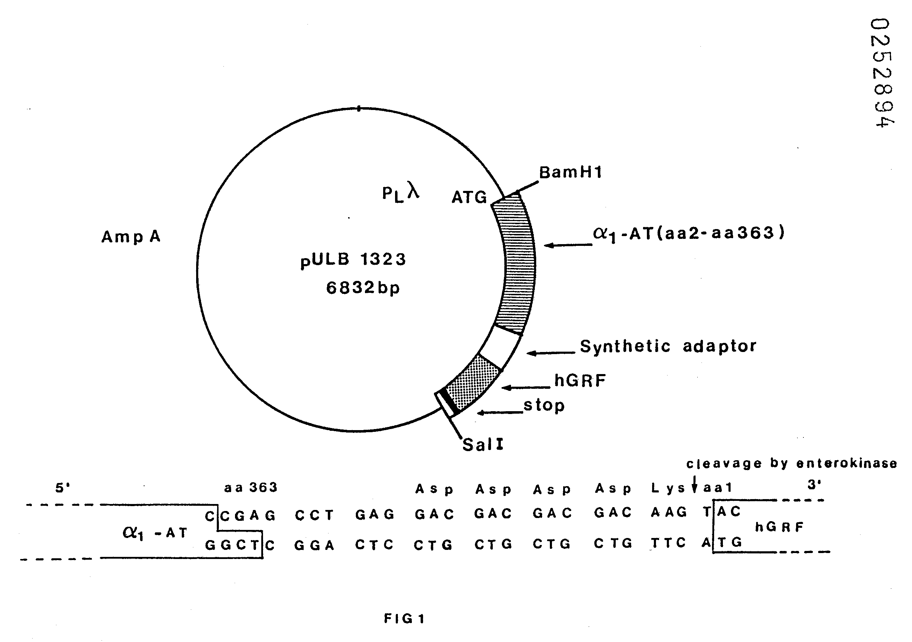

Stade 3. L'adaptateur ADN de synthèse représenté dans la Figure 1 est obtenu par la technique décrite par CRAVADOR et al. (Biochimie 67, 829, 1985). Les deux fragments mono-brins, respectivement de 27 bp et 23 bp, lorsqu'ils sont hybridés (annealed) comme décrit par CRAVADOR et al. (loc. cit.) génèrent un fragment d'ADN double brin portant une extrémité cohésive 5'Aval et une extrémité cohésive 3'Scal. Ces extrémités cohésives conviennent pour la fusion en phase du fragment BamHI-Aval d'hAT et du fragment Rsal-Sall d'hGRF. De plus, l'adaptateur synthétique code pour la séquence d'acides aminés (Asp)$_4$Lys qui est clivable in vitro par l'enzyme entérokinase comme montré par MAROUX et al. (loc. cit.).

Stade 4. L'ADN d'un vecteur d'expression plasmidique (p. ex. pAS1 décrit par ROSENBERG et al. dans Methods in Enzymology 101, 123-138, 1983) est clivé avec les enzymes BamHI et Sall. Le grand fragment résultant de la digestion est retenu; il porte le puissant promoteur P$_L$ (lambda) et un codon d'initiation ATG.

Les fragments ADN des stades 1 à 4 sont assemblés par ligation en utilisant des procédures standard (MANIATIS et al. Molecular cloning : a laboratory manual, Cold Spring Harbour Laboratory, New-York, 1982). La construction résultante (pULB1323) est un vecteur d'expression qui contient le fragment du gène hAT fusionné en phase au gène hGRF via un adaptateur synthétique (Fig. 1). La partie supérieure de la Figure 1 représente le plasmide recombinant pULB1323 qui porte les séquences codant pour hAT, l'adaptateur synthétique et hGRF fusionnées. La partie inférieure de la Figure 1 représente la séquence des nucléotides et des acides aminés de l'adaptateur synthétique.

Une souche lysogène de E. coli (p. ex. la souche AR58 décrite par MOTT et al. (Proc. Natl. Acad. Sci. USA 82, 88-92, 1985) est transformée avec pULB1323 en utilisant les procédés standard (MANIATIS et al., loc. cit.) et les transformants sont cultivés jusqu'à la phase exponentielle avant induction thermique (M. ROSENBERG et al., Meth. Enzymol. 101, 123, 1983).

Après la période d'induction, les cellules sont rassemblées par centrifugation et on réalise leur lyse par addition d'une solution de lysozyme (1 g de cellules par 10 ml de solution) 50 mM Tris pH 8; 0,1 mM éthylènediaminetétraacetate de sodium (EDTA); 0,1 mM dithiothréitol (DTT); 0,1 % Triton X100; 25 % saccharose et 0,5 mg/ml de lysozyme. La suspension est alors séparée par centrifugation à vitesse moyenne (20.000 g) pendant 20 minutes.

Des échantillons du polypeptide hybride hAT/hGRF ont été analysés sur gel de polyacrylamide dodecyl sulfate (LAEMLY, Nature 227, 680, 1970) et identifiés par Western blot et immunodétection (BOLLEN et al., DNA 2, 255, 1983).

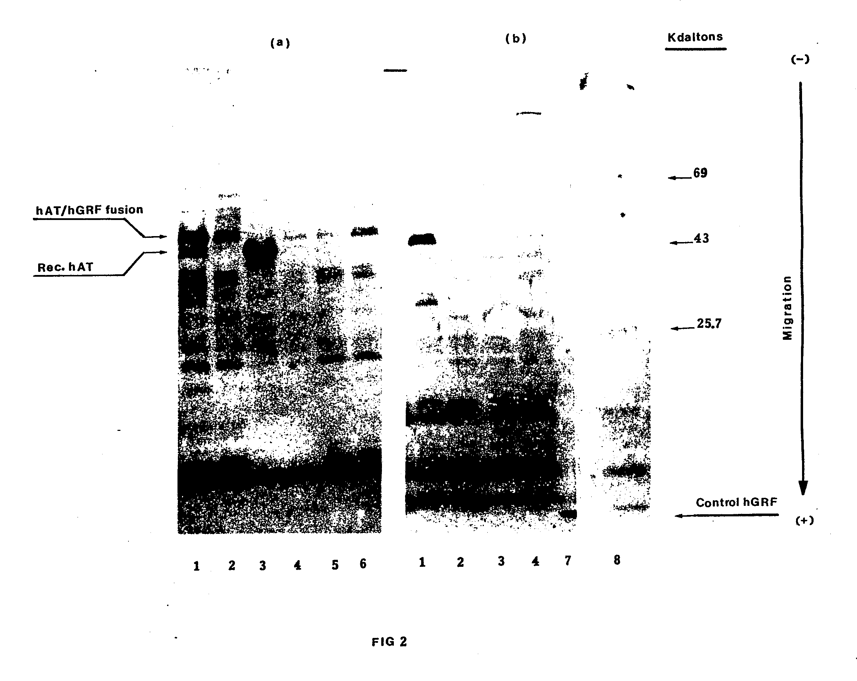

Comme le montre la Figure 2, le polypeptide hybride hAT/hGRF codé par pULB1323 peut être détecté (bande 1) à l'aide d'anticorps soit anti-hAT (panneau A) soit anti-hGRF (panneau b) et ceci contraste fortement avec les essais tendant à l'expression d'hGRF mature telle quelle dans E. coli dans lesquels aucun niveau de détection ne peut être observé (CRAVADOR, loc. cit.). De plus, le polypeptide hybride a le poids moléculaire attendu pour une fusion entre hAT (de aa$_2$ jusqu'à aa$_{363}$), la séquence d'acides aminés reconnue par l'entérokinase et hGRF mature. Des contrôles appropriés ont été utilisés au cours de l'expérience, à savoir des échantillons de pULB1323 non induit (bande 2), recombinant hAT induit (bande 3); idem, non induit (bande 4); témoin négatif pAS1 (bandes 5 et 6); hGRF standard (bande 7) et poids moléculaire standard (bande 8).

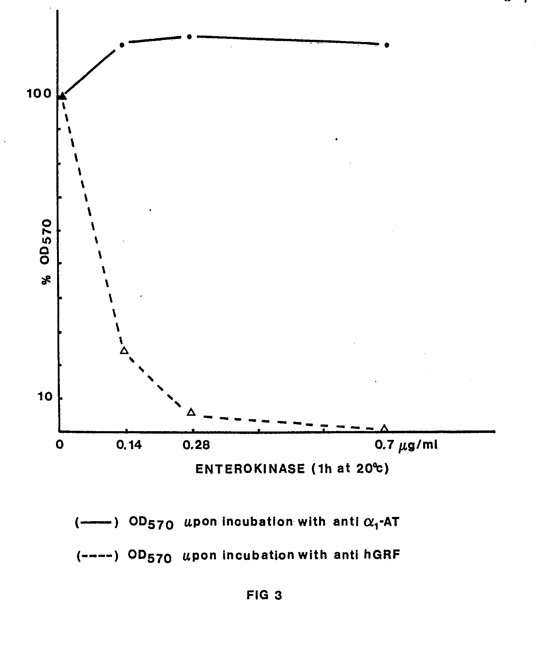

Des échantillons de polypeptide hybride hAT/hGRF ont été mis à digérer in vitro avec de l'entérokinase de manière à cliver la fusion hAT/hGRF et à libérer hGRF mature. La Figure 3 représente ce clivage. L'essai consiste en un ELISA; il montre que hGRF est libéré du polypeptide hybride qui a été lié par son fragment hAT à des plaques de cupules chargées d'anticorps anti hAT (Δ--Δ). La Figure montre également que l'hAT lié n'est pas libéré des microcupules chargées pendant l'essai (. - .).

Si on le désire, l'hGRF mature clivée peut alors encore être davantage purifié par chromatographie d'affinité dans des conditions bien connues dans la pratique ou par d'autres techniques d'isolement de protéines.

## Revendications

1. Molécule d'ADN comprenant une fusion en phase et lue de l'extrémité 5' à l'extrémité 3' d'une séquence codante d'hAT, un adaptateur de synthèse codant pour une séquence d'acides aminés clivable in vitro et le gène de hGRF ou un fragment ou dérivé actif de celui-ci.

2. Molécule d'ADN de la revendication 1 dans laquelle la séquence codante d'hAT est un fragment de la séquence codant pour hAT et s'étendant du codon pour l'acide aminé 2 environ ou l'acide aminé 15 environ jusqu'à l'acide aminé 363 environ.

3. Molécule d'ADN de la revendication 1 dans laquelle le gène hGRF ou le fragment ou dérivé actif de celui-ci est entièrement synthétique.

4. Molécule d'ADN de la revendication 1 dans laquelle l'adaptateur synthétique code pour la séquence d'acides aminés (Asp)$_4$Lys.

5. Molécule d'ADN suivant l'une quelconque des revendications 1 à 4 caractérisée en ce qu'elle est un vecteur d'expression bactérien dans lequel la fusion est insérée de manière opérative.

6. Molécule d'ADN de la revendication 5 caractérisée en ce qu'elle est un vecteur d'expression d'E. coli

7. Molécule d'ADN de la revendication 6 dans laquelle le vecteur d'expression comprend la région régulatrice de pAS1.

8. Polypeptide hybride comprenant hAT et hGRF ou un fragment ou dérivé actif de celui-ci pontés par la séquence d'acides aminés (Asp)$_4$Lys clivable in vitro.

9. Polypeptide hybride de la revendication 8 dans lequel hAT est le fragment de hAT s'étendant de l'acide aminé 2 environ ou de l'acide aminé 15 environ jusqu'à l'acide aminé 363 environ.

10. Polypeptide hybride de la revendication 8 dans lequel hAT est le fragment de hAT s'étendant de l'acide aminé 2 ou 15 jusqu'à l'acide aminé 363.

11. Bactérie transformée avec la molécule d'ADN de l'une quelconque des revendications 1 à 4.

12. Bactérie de la revendication 11 caractérisée en ce qu'elle est un E. coli.

13. Bactérie transformée avec une molécule d'ADN suivant la revendication 5.

14. Bactérie de la revendication 13 caractérisée en ce qu'elle est un E. coli.

15. E. coli transformé avec une molécule d'ADN suivant la revendication 6.

16. E. coli transformé avec une molécule d'ADN suivant la revendication 7.

17. Procédé de production d'un polypeptide hybride caractérisée en ce qu'on cultive une bactérie suivant l'une quelconque des revendications 11 à 16 dans des conditions permissives et qu'on isole le polypeptide hybride de la culture.

18. Méthode de production de hGRF caractérisée en ce qu'on clive avec une enzyme entérokinase un polypeptide suivant l'une quelconque des revendications 8 à 10.

Revendications pour les Etats Contractants AT, ES, GR

1. Procédé de production d'un polypeptide hybride caractérisé en ce qu'il comprend la culture en conditions permissives d'une bactérie transformée avec une molécule d'ADN comprenant la fusion en phase et lue de l'extrémité 5' à l'extrémité 3', une séquence codante de hAT, un adaptateur de synthèse codant pour une séquence d'acides aminés clivable in vitro et le gène de hGRF ou un fragment ou dérivé actif de celui-ci et l'isolement du polypeptide hybride de ladite culture.

2. Procédé suivant la revendication 1 dans lequel la séquence codante de hAT est un fragment de la séquence codante pour hAT s'étendant du codon pour l'acide aminé 2 environ ou pour l'acide aminé 15 environ jusqu'à l'acide aminé 363 environ.

3. Procédé suivant la revendication 1 dans lequel le gène de hGRF ou son fragment ou dérivé actif est entièrement synthétique.

4. Procédé suivant la revendication 1 dans lequel l'adaptateur synthétique code pour la séquence d'acides aminés (Asp)$_4$Lys.

5. Procédé suivant l'une quelconque des revendications 1 à 4 dans lequel la molécule d'ADN est un vecteur d'expression bactérien dans lequel la fusion est insérée de manière opérative.

6. Procédé suivant la revendication 5 dans lequel le vecteur d'expression est un vecteur d'expression de E. coli.

7. Procédé suivant la revendication 6 dans lequel le vecteur d'expression comprend la région régulatrice de pAS1.

8. Méthode de production de hGRF caractérisée en ce qu'elle comprend le clivage par une enzyme entérokinase d'un polypeptide hybride préparé par une méthode suivant l'une quelconque des revendications 1 à 7.

FIG 1

0252894

FIG 2

(b)

(a)

Migration

(−) → (+)

Kdaltons

69

43

25.7

Control hGRF

8  7  3  4  1  2  1

1  2  3  4  5  6

hAT/hGRF fusion

Rec. hAT

(———) OD$_{570}$ upon incubation with anti $\alpha_1$-AT

(- - - -) OD$_{570}$ upon incubation with anti hGRF

FIG 3